# EUROPEAN PATENT APPLICATION

(11) **EP 3 677 202 A1**
(43) Date of publication of application: **08.07.2020**
(21) Application number: 19218663.3
(22) Date of filing: 20.12.2019
(51) Int. Cl.: A61B 17/34

(54) **SURGICAL ACCESS DEVICE AND SLEEVE STOPS FOR USE THEREWITH**

(30) Priority: 02.01.2019 US 201916237788
(71) Applicant: Covidien LP, Mansfield, MA 02048 (US)
(72) Inventor: AVVLN, Srinivasa Murthy Aravalli, 500049 Hyderabad (IN)
(74) Representative: Maschio, Antonio

(57) **Abstract**

A surgical access device includes a cannula (200) and a sleeve stop (500). The sleeve stop is configured for selective engagement with an elongated portion (220) of the cannula, and configured to limit distal advancement of the cannula with respect to tissue (T). The sleeve stop is a unitary structure and includes a body portion having a first section and a second section. The first section and the second section are interconnected by a living hinge. The first section is configured to selectively engage the second section.

## Description

### TECHNICAL FIELD

The present disclosure relates to a surgical access device. More particularly, the present disclosure relates to sleeve stops for use with a surgical access device.

### BACKGROUND OF RELATED ART

Endoscopic and laparoscopic minimally invasive procedures have been used for introducing medical devices inside a patient and for viewing portions of the patient's anatomy. To view a desired anatomical site, a surgeon may insert a rigid or flexible endoscope inside the patient to render images of the anatomical site.

Typically, a trocar assembly includes a cannula and an obturator. The cannula remains in place for use during the laparoscopic procedure, and the obturator includes a tip for penetrating body tissue. In endoscopic surgical procedures, surgery is performed in any hollow organ or tissue of the body through a small incision or through a narrow endoscopic tube (e.g., a cannula) inserted through a small entrance wound in the skin. In laparoscopic procedures, surgical operations in the abdomen are performed through small incisions (usually about 0.5 to about 1.5 cm). Laparoscopic and endoscopic procedures often require the surgeon to act on organs, tissues and vessels far removed from the incision.

Accordingly, it may be helpful to provide a sleeve stop that is usable with trocar assemblies and is configured to help prevent over-insertion or over-travel of the trocar assembly within a surgical site.

### SUMMARY

The present disclosure relates to a surgical access device including a cannula and a sleeve stop. The cannula includes a housing and an elongated portion extending distally from the housing. The elongated portion defines a channel extending therethrough. A distal portion of the elongated portion is configured for engaging tissue. The sleeve stop is configured for selective engagement with the elongated portion of the cannula and is configured to limit distal advancement of the cannula with respect to tissue. The sleeve stop is a unitary structure and includes a body portion having a first section and a second section. The first section and the second section are interconnected by a living hinge. The first section is configured to selectively engage the second section.

In disclosed embodiments, the first section of the body portion of the sleeve stop may include a finger, and the second section of the body portion of the sleeve stop may include a receptacle configured for slidingly receiving the finger. It is further disclosed that the finger and the receptacle may include a plurality of teeth. In embodiments, engagement between the plurality of teeth of the finger and the plurality of teeth of the receptacle may help maintain a size of an aperture defined between the first section of the body portion of the sleeve stop and the second portion of the body portion of the sleeve stop.

It is also disclosed that the sleeve stop may be made from a single material, such as plastic.

It is further disclosed that the surgical access device may include a stopping block positionable on the elongated portion of the cannula and distally of the sleeve stop. The stopping block may be configured to contact tissue and to prevent the sleeve stop from contacting tissue.

The present disclosure also relates to a surgical access device including a cannula and a sleeve stop. The cannula includes a housing and an elongated portion extending distally from the housing. The elongated portion defines a channel extending therethrough, and a distal portion of the elongated portion is configured for engaging tissue. The sleeve stop is configured for selective engagement with the elongated portion of the cannula, and is configured to limit distal advancement of the cannula with respect to tissue. The sleeve stop includes an adjustable member and a body portion having a first section and a second section. The first section is pivotable relative to the second section about a pivot. The first section and the second section define an aperture therebetween for slidable reception of the cannula. The adjustable member is configured to threadably engage a threaded aperture of the first section of the sleeve stop and a threaded aperture of the second section of the sleeve stop.

In disclosed embodiments, rotation of the adjustable member in a first direction relative to the first section of the sleeve stop may cause a diameter of the aperture to increase, and rotation of the adjustable member in a second direction relative to the first section of the sleeve stop may cause the diameter of the aperture to decrease.

It is also disclosed that the adjustable member may include a wing nut.

It is further disclosed that the first section of the sleeve stop may include a semi-circular portion and an extension portion, and the second section of the sleeve stop may include a semi-circular portion and an extension portion. In embodiments, the threaded aperture of the first section of the sleeve stop may be on the extension of the first section, and the threaded aperture of the second section of the sleeve stop may be on the extension of the second section.

Additionally, it is disclosed that the surgical access device may include a stopping block positionable on the elongated portion of the cannula and distal of the sleeve stop. The stopping block may be configured to contact tissue and to prevent the sleeve stop from contacting tissue.

### BRIEF DESCRIPTION OF THE DRAWINGS

Various embodiments of the present disclosure are illustrated herein with reference to the accompanying drawings, wherein:
FIG. 1 is a perspective view of a trocar assembly including a cannula and an obturator;
FIG. 2 is a perspective view of a first embodiment of a sleeve stop for use with the cannula of FIG. 1;
FIG. 3 is an assembly view of the trocar assembly of FIG. 1, the first embodiment of the sleeve stop of FIG. 2, and a stopping block;
FIG. 4 is a perspective view of the trocar assembly of FIGS. 1 and 3 including the first embodiment of the sleeve stop of FIGS. 2 and 3 and the stopping block of FIG. 3 engaged with tissue;
FIG. 5 is a top view of a second embodiment of a sleeve stop;
FIG. 6 is a perspective view of a third embodiment of a sleeve stop;
FIG. 7 is a side view of the third embodiment of the sleeve stop of FIG. 6;
FIGS. 8 and 9 are side views of a fourth embodiment of a sleeve stop; and
FIGS. 10 and 11 are perspective views of a fifth embodiment of a sleeve stop.

### DETAILED DESCRIPTION

Embodiments of the presently disclosed surgical access device and sleeve stops are described in detail with reference to the drawings, wherein like reference numerals designate corresponding elements in each of the several views.

As used herein, the term "distal" refers to that portion of the instrument, or component thereof which is farther from the user while the term "proximal" refers to that portion of the instrument or component thereof which is closer to the user.

Various embodiments of a surgical access device are described herein. Generally, the surgical access device includes a trocar assembly which may be employed during surgery (e.g., laparoscopic surgery) and may, in various embodiments, provide for the sealed access of laparoscopic surgical instruments into an insufflated body cavity, such as the abdominal cavity. As will be described in additional detail below, the trocar assemblies of the present disclosure include a cannula and an obturator insertable therethrough. The cannula and obturator are separate components but are capable of being selectively connected together. For example, the obturator may be inserted into and through the cannula until the handle of the obturator engages, e.g., selectively locks into, a proximal housing of the cannula. In this initial position, the trocar assembly is employed to tunnel through an anatomical structure, e.g., the abdominal wall, either by making a new passage through the structure or by passing through an existing opening through the structure. Once the trocar assembly has tunneled through the anatomical structure, the obturator is removed, leaving the cannula in place in the structure, e.g., in the incision created by the trocar assembly. The proximal housing of the cannula may include seals or valves that prevent the escape of insufflation gases from the body cavity, while also allowing surgical instruments to be inserted into the body cavity. Further details of a surgical access device including a cannula and an obturator are described in U.S. Patent No. 10,022,149 to Holsten et al., issued on July 17, 2018, and U.S. Patent Application Publication No. 2018/0085145 to Okoniewski et al., filed on November 13, 2017, the entire content of each of which being incorporated by reference herein.

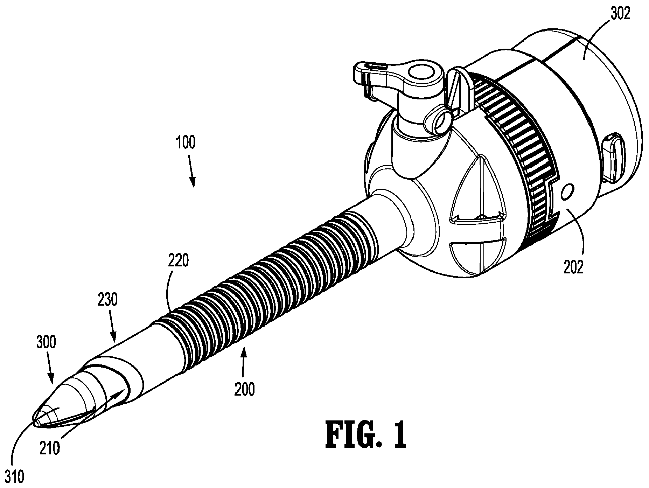

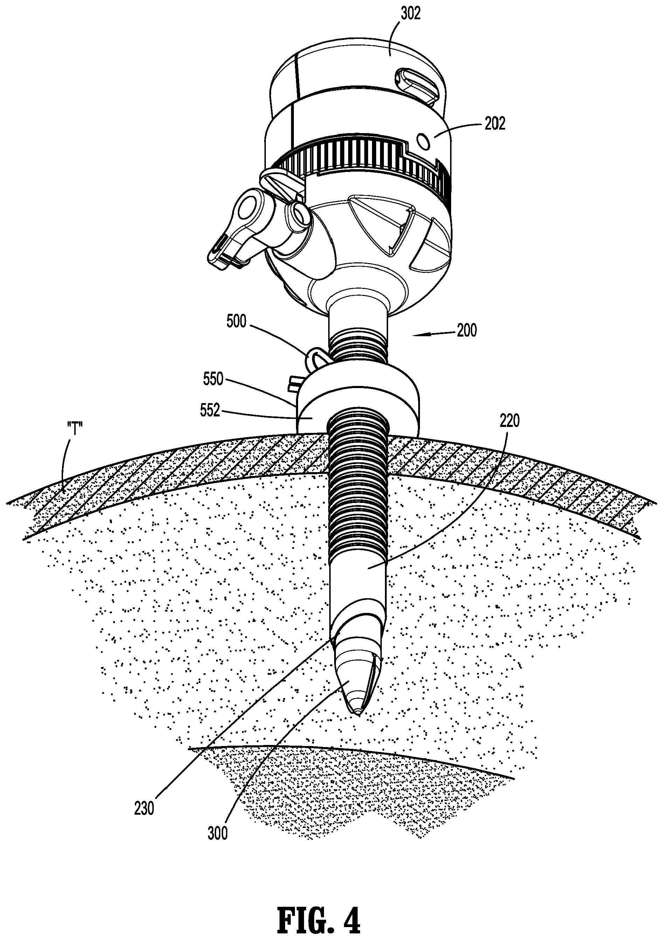

With initial reference to FIG. 1, a surgical access device 100 is shown. Surgical access device 100 includes a cannula 200, and an obturator 300. The obturator 300 is insertable through a channel 210 defined by an elongated portion 220 of cannula 200. Additionally, obturator 300 is selectively engageable with or attachable to cannula 200. More particularly, a proximal portion 302 of obturator 300 is selectively engageable with or attachable to a proximal portion or housing 202 of cannula 200. In use, when obturator 300 is engaged with cannula 200, a distal end 310 of obturator 300 is advanced into tissue "T" to create or enlarge an incision or opening in tissue "T" (FIG. 4). Alternatively, a distal end 230 of cannula 200 can be used to create or enlarge an opening in tissue "T," without the use of an obturator, for instance. In either situation, it may be important to limit or control the insertion depth of cannula 200 with respect to the tissue "T" to help provide optimal access to the target tissue, and to minimize accidental contact between portions of cannula 200 or obturator 300 with tissue located distally of the target tissue. The present disclosure includes various embodiments of sleeve stops that are positionable on cannula 200, and which are configured to limit the distance cannula 200 can be advanced with respect to the tissue "T."

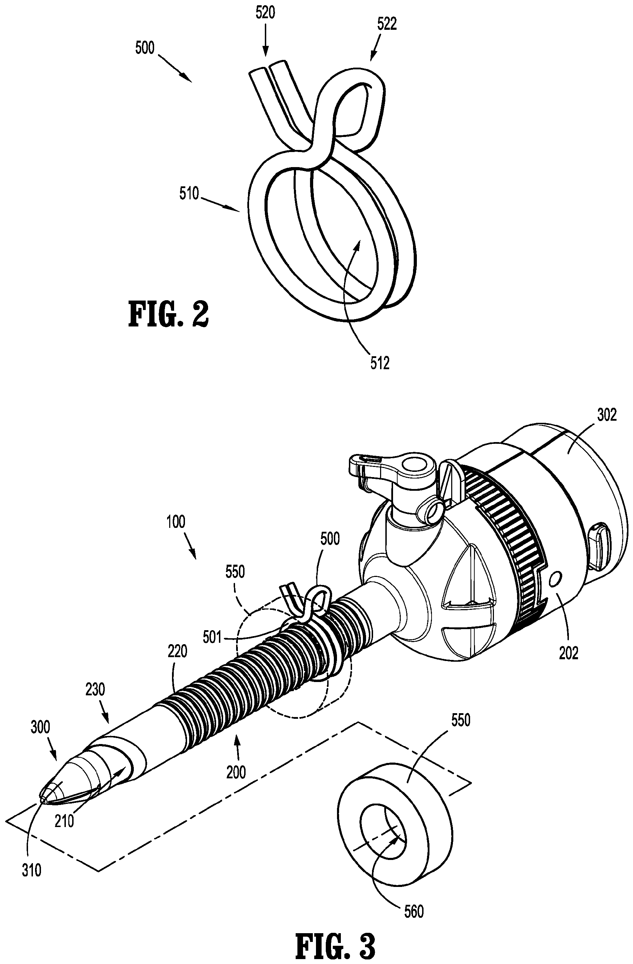

With particular reference to FIGS. 2-4, a first embodiment of a sleeve stop is shown and is indicated by reference character 500. Sleeve stop 500 is a spring-loaded clamp, and includes a ring portion 510, and two extensions 520, 522. Ring portion 510 defines an aperture 512 therethrough which is sized to engage an outer circumference of elongated portion 220 of cannula 200. Extensions 520, 522 extend from and are an integral part of ring portion 510. In embodiments, ring portion 510 and extensions 520, 522 are a unitary structure. As shown, sleeve stop 500 may be formed of a single wire formed into the illustrated shape or a similar shape.

When extensions 520, 522 are moved toward one another (e.g., by squeezing them together), the diameter of aperture 512 changes from a first, smaller diameter to a second, larger diameter. Extensions 520, 522 are biased (e.g., spring-loaded) away from each other, thereby biasing aperture 512 towards its first, smaller diameter.

In its initial, biased position, aperture 512, defined by ring portion 510 of sleeve stop 500, is configured to fixedly or non-slidingly engage elongated portion 220 of cannula 200. In its second, non-biased position, aperture 512 is at least slightly larger than an outer diameter of elongated portion 220 of cannula 200 thereby enabling sleeve stop 500 to be repositioned along elongated portion 220 of cannula 200..

Additionally, as shown in FIGS. 3 and 4, a stopping block 550 is also positionable on elongated portion 220 of cannula 200, and distal of sleeve stop 500. Stopping block 550 is configured to contact tissue, and is configured to prevent sleeve stop 500 from contacting tissue, for example. Stopping block 550 includes an aperture 560 having a larger diameter than an outer diameter of elongated portion 220 of cannula 200, thereby allowing stopping block 550 to slidingly engage elongated portion 220 of cannula 200.

In use, a user moves extensions 520, 522 of sleeve stop 500 toward each other to enlarge aperture 512, slides sleeve stop 500 to a desired position along elongated portion 220 of cannula 200, and releases extensions 520, 522 to decrease the size of aperture 512 such that sleeve stop 500 is fixedly or non-slidingly positioned on elongated portion 220 of cannula 200. With particular reference to FIGS. 3 and 4, when distal end 230 of cannula 200 (and/or obturator 300) is inserted into tissue "T," a distal face 552 of stopping block 550 contacts the tissue "T," and a distal face 501 of sleeve stop 500 contacts a proximal face (not visible in FIGS. 3 or 4) of stopping block 550. This engagement between the tissue "T" and stopping block 550, and between stopping block 500 and sleeve stop 500 helps prevent further insertion of cannula 200 with respect to the tissue "T." Following the surgical procedure, for instance, sleeve stop 500 and/or stopping block 550 can be removed from cannula 200, sterilized, and re-used.

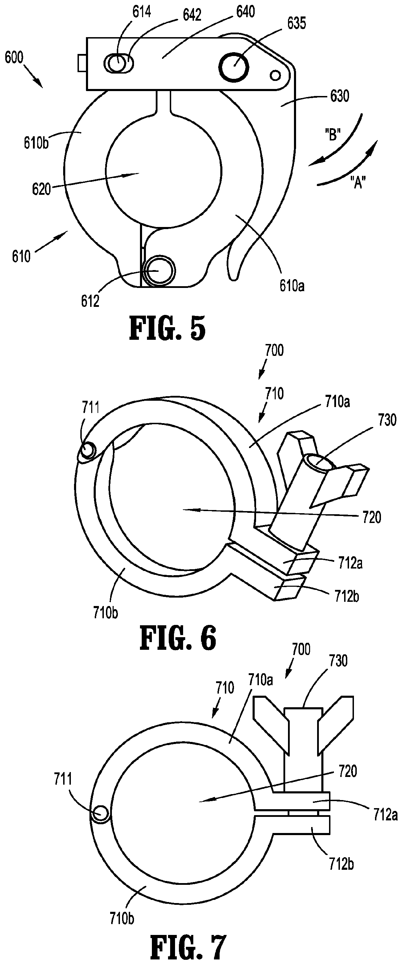

Referring now to FIG. 5, a second embodiment of a sleeve stop is shown and is indicated by reference character 600. Sleeve stop 600 is configured to engage elongated portion 220 of cannula 200, and is usable with stopping block 550, as discussed above. Sleeve stop 600 includes a body portion 610 defining an aperture 620, a lever 630, and a link 640 interconnecting body portion 610 and lever 630. Body portion 610 includes a first section 610a that is pivotably connected to a second section 610b with a first pivot 612. Together, first section 610a and second section 610b define aperture 620 therebetween, which is sized to engage an outer circumference of elongated portion 220 of cannula 200.

Link 640 includes a slot 642 configured to slidingly engage a pin 614 of second section 610b of body portion 610. Link 640 is pivotably engaged with lever 630 by a second pivot 635. Lever 630 is pivotable about second pivot 635 in the general direction of arrow "A" in FIG. 5 when transitioning from a first position (shown) to a second position (not shown), and in the general direction of arrow "B" in FIG. 5 when transitioning from the second position to the first position. As lever 630 pivots, link 640 slides relative to second section 610b of body portion 610 (i.e., pin 614 of second section 610b slides within slot 642 of link 640), such that the diameter defined by aperture 620 changes. When lever 630 is in its first position (shown), the diameter defined by aperture 620 is relatively small such that sleeve stop 600 is configured to fixedly or non-slidingly engage elongated portion 220 of cannula 200. When lever 640 is in its second position, the diameter defined by aperture 620 is relatively large (i.e., larger than an outer diameter of elongated portion 220 of cannula 200) such that sleeve stop 600 is configured to be repositioned along elongated portion 220 of cannula 200.

In use, a user moves lever 630 of sleeve stop 600 in the general direction of arrow "A" away from first section 610a of body portion 610 thereby enlarging aperture 620, slides or repositions sleeve stop 600 to a desired position along elongated portion 220 of cannula 200, and moves lever 630 in the general direction of arrow "B" toward first section 610a of body portion 610 to decrease the size of aperture 620 such that sleeve stop 600 is fixedly or non-slidingly positioned on elongated portion 220 of cannula 200. Sleeve stop 600 is also usable with stopping block 550 in a similar manner as shown herein and described above with regard to sleeve stop 500. Accordingly, the use of sleeve stop 600 helps prevent further insertion of cannula 200 with respect to the tissue "T." Following the surgical procedure, for instance, sleeve stop 600 and/or stopping block 550 can be removed from cannula 200, sterilized, and re-used.

Referring now to FIGS. 6 and 7, a third embodiment of a sleeve stop is shown and is indicated by reference character 700. Sleeve stop 700 is configured to engage elongated portion 220 of cannula 200, and is usable with stopping block 550, as discussed above. Sleeve stop 700 includes a body portion 710 having a first section 710a and a second section 710b, which are pivotable with respect to each other about pivot 711, and which together define an aperture 720 that is sized to engage an outer circumference of elongated portion 220 of cannula 200. Sleeve stop 700 also includes an adjustable member 730 (e.g., a wing nut) rotatably engageable with first section 710a and second section 710b of body portion 710.

Adjustable member 730 includes a threaded portion (hidden from view in the figures), which is configured to engage corresponding threaded sections of respective extensions 712a, 712b of first section 710a and second section 710b of body portion 710. As adjustable member 730 is rotated in a first direction (e.g., clockwise) relative to body portion 710, extension 712a of first section 710a moves toward extension 712b of second section 710b (about pivot 711), thereby reducing the size of aperture 720 such that sleeve stop 700 fixedly or non-slidingly engages elongated portion 220 of cannula 200 and is unable to slide relative thereto. As adjustable member 730 is rotated in a second direction (e.g., counter-clockwise) relative to body portion 710, extension 712a of first section 710a moves away from extension 712b of second section 710b (about pivot 711), thereby increasing the size of aperture 720 such that sleeve stop 700 is repositionable along elongated portion 220 of cannula 200.

In use, a user rotates adjustable member 730 of sleeve stop 700 in the second direction, for instance, to thereby enlarge aperture 720, slides sleeve stop 700 to a desired position along elongated portion 220 of cannula 200, and rotates adjustable member 730 in the first, opposite direction, for instance, to decrease the size of aperture 720 such that sleeve stop 700 is fixedly or non-slidingly positioned on elongated portion 220 of cannula 200. Sleeve stop 700 is also usable with stopping block 550 in a similar manner as shown herein and described above with regard to sleeve stop 500. Accordingly, the use of sleeve stop 700 helps prevent further insertion of cannula 200 with respect to the tissue "T." Following the surgical procedure, for instance, sleeve stop 700 and/or stopping block 550 can be removed from cannula 200, sterilized, and re-used.

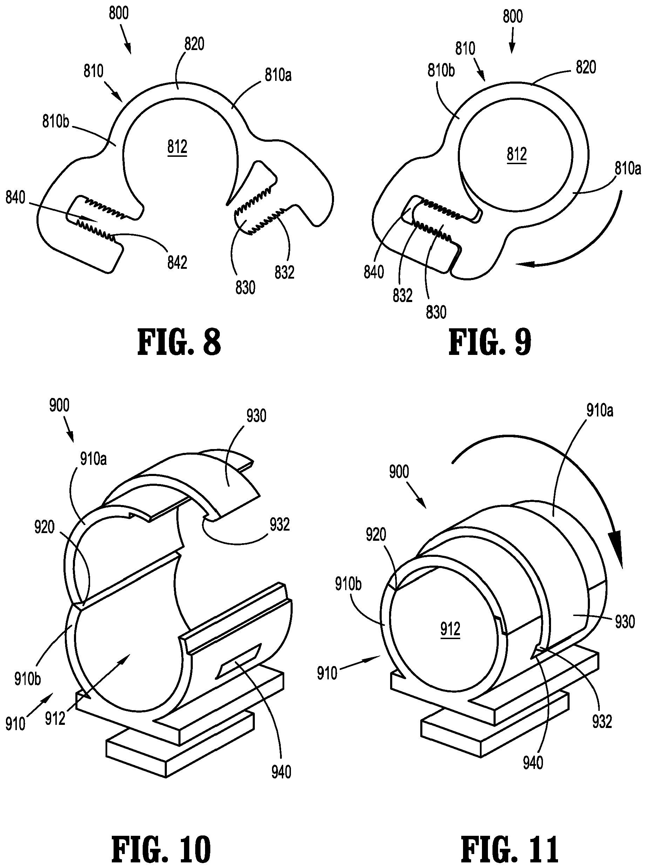

Referring now to FIGS. 8-9, a fourth embodiment of a sleeve stop is shown and is indicated by reference character 800. Sleeve stop 800 is configured to engage elongated portion 220 of cannula 200, and is usable with stopping block 550, as discussed above. Sleeve stop 800 is a unitary structure and includes a body portion 810 having a first section 810a and a second section 810b interconnected by a living hinge 820. It is envisioned that an entirety of sleeve stop 800 is made from a single material, e.g., plastic. Together, first section 810a and second section 810b define an aperture 812. First section 810a of body portion 810 includes a finger 830 having a plurality of teeth 832. Second section 810b of body portion 810 includes a receptacle 840 having a plurality of teeth 842 for slidingly receiving finger 830.

Engagement between plurality of teeth 832 of finger 830 and plurality of teeth 842 of receptacle helps maintain a desired portion of finger 830 within receptacle 840, which corresponds to a desired size of aperture 812. When aperture 812 is larger than an outer diameter of elongated portion 220 of cannula 200 (e.g., FIG. 8), sleeve stop 800 is able to be repositionable along elongated portion 220 of cannula 200. When aperture 812 is equal to or smaller than the outer diameter of elongated portion 220 of cannula 200 (e.g., FIG. 9), sleeve stop 800 fixedly engages elongated portion 220 of cannula 200 and is unable to slide relative thereto.

In use, a user is able to position sleeve stop 800 around elongated portion 220 of cannula 200 when sleeve stop 800 is in an open position (FIG. 8), slide sleeve stop 800 to a desired position along elongated portion 220 of cannula 200, and insert at least a portion of finger 830 into receptacle 840 to decrease the size of aperture 812 and to maintain the position of first section 810a of body portion 810 relative to second section 810b of body portion 810 (FIG. 9) such that sleeve stop 800 is fixedly or non-slidingly positioned on elongated portion 220 of cannula 200. Sleeve stop 800 is also usable with stopping block 550 in a similar manner as shown herein and described above with regard to sleeve stop 500. Accordingly, the use of sleeve stop 800 helps prevent further insertion of cannula 200 with respect to the tissue. Following the surgical procedure, for instance, sleeve stop 800 and/or stopping block 550 can be removed from cannula 200, sterilized, and re-used.

Referring now to FIGS. 10-11, a fifth embodiment of a sleeve stop is shown and is indicated by reference character 900. Sleeve stop 900 is configured to engage elongated portion 220 of cannula 200, and is usable with stopping block 550, as discussed above. Sleeve stop 900 includes a body portion 910 having a first section 910a and a second section 910b interconnected by a living hinge 920. Together, first section 910a and second section 910b define an aperture 912. First section 910a of body portion 910 includes a finger 930 having a lip 932. Second section 910b of body portion 910 includes a receptacle 940 for receiving lip 932 of finger 930.

Engagement between lip 932 of finger 930 and receptacle 940 helps maintain first section 910a and second section 910b of body portion 910 in a closed position (FIG. 11), which corresponds to a desired size of aperture 912. When first section 910a and second section 910b of body portion 910 are in an open position (FIG. 10), aperture 912 is larger than an outer diameter of elongated portion 220 of cannula 200 such that sleeve stop 900 is able to be repositionable along elongated portion 220 of cannula 200. When first section 910a and second section 910b of body portion 910 are a closed position (FIG. 11), aperture 912 is equal to or smaller than the outer diameter of elongated portion 220 of cannula 200 such that sleeve stop 900 fixedly engages elongated portion 220 of cannula 200 and is unable to slide relative thereto.

In use, a user is able to position sleeve stop 900 around elongated portion 220 of cannula 200 when sleeve stop 900 is in an open position (FIG. 10), slide sleeve stop 900 to a desired position along elongated portion 220 of cannula 200, and insert lip 932 of finger 930 into receptacle 940 to decrease the size of aperture 912 and to maintain the position of first section 910a of body portion 910 relative to second section 910b of body portion 910 (FIG. 11) such that sleeve stop 900 is fixedly or non-slidingly positioned on elongated portion 220 of cannula 200. Sleeve stop 900 is also usable with stopping block 550 in a similar manner as shown herein and described above with regard to sleeve stop 500. Accordingly, the use of sleeve stop 900 helps prevent further insertion of cannula 200 with respect to the tissue. Following the surgical procedure, for instance, sleeve stop 900 and/or stopping block 550 can be removed from cannula 200, sterilized, and re-used.

While the above description contains many specifics, these specifics should not be construed as limitations on the scope of the present disclosure, but merely as illustrations of various embodiments thereof. Therefore, the above description should not be construed as limiting, but merely as exemplifications of various embodiments. Those skilled in the art will envision other modifications within the scope and spirit of the claims appended hereto.

The invention may be described by reference to the following numbered paragraphs:
1. A surgical access device comprising: a cannula having a housing and an elongated portion extending distally from the housing, the elongated portion defining a channel extending therethrough, a distal portion of the elongated portion configured for engaging tissue; and a sleeve stop configured for selective engagement with the elongated portion of the cannula, and configured to limit distal advancement of the cannula with respect to tissue, the sleeve stop being a unitary structure and including a body portion having a first section and a second section, the first section and the second section are interconnected by a living hinge, wherein the first section is configured to selectively engage the second section.
2. The surgical access device according to paragraph 1, wherein the first section of the body portion of the sleeve stop includes a finger, and wherein the second section of the body portion of the sleeve stop includes a receptacle configured for slidingly receiving the finger.
3. The surgical access device according to paragraph 2, wherein the finger includes a plurality of teeth.
4. The surgical access device according to paragraph 3, wherein the receptacle includes a plurality of teeth.
5. The surgical access device according to paragraph 4, wherein engagement between the plurality of teeth of the finger and the plurality of teeth of the receptacle helps maintain a size of an aperture defined between the first section of the body portion of the sleeve stop and the second portion of the body portion of the sleeve stop.
6. The surgical access device according to paragraph 1, wherein engagement between the first section of the body portion of the sleeve stop and the second portion of the body portion of the sleeve stop defines an aperture therebetween.
7. The surgical access device according to paragraph 1, wherein the sleeve stop is made from a single material.
8. The surgical access device according to paragraph 7, wherein the sleeve stop is made from plastic.
9. The surgical access device according to paragraph 1, further including a stopping block positionable on the elongated portion of the cannula and distally of the sleeve stop, the stopping block configured to contact tissue and to prevent the sleeve stop from contacting tissue.
10. A surgical access device comprising: a cannula having a housing and an elongated portion extending distally from the housing, the elongated portion defining a channel extending therethrough, a distal portion of the elongated portion configured for engaging tissue; and a sleeve stop configured for selective engagement with the elongated portion of the cannula, and configured to limit distal advancement of the cannula with respect to tissue, the sleeve stop including an adjustable member and a body portion having a first section and a second section, the first section being pivotable relative to the second section about a pivot, the first section and the second section defining an aperture therebetween for slidable reception of the cannula, the adjustable member configured to threadably engage a threaded aperture of the first section of the sleeve stop and a threaded aperture of the second section of the sleeve stop.
11. The surgical access device according to paragraph 10, wherein rotation of the adjustable member in a first direction relative to the first section of the sleeve stop causes a diameter of the aperture to increase, and wherein rotation of the adjustable member in a second direction relative to the first section of the sleeve stop causes the diameter of the aperture to decrease.
12. The surgical access device according to paragraph 10, wherein the adjustable member includes a wing nut.
13. The surgical access device according to paragraph 10, wherein the first section of the sleeve stop includes a semi-circular portion and an extension portion, and wherein the second section of the sleeve stop includes a semi-circular portion and an extension portion.
14. The surgical access device according to paragraph 13, wherein the threaded aperture of the first section of the sleeve stop is on the extension of the first section, and wherein the threaded aperture of the second section of the sleeve stop is on the extension of the second section.
15. The surgical access device according to paragraph 10, further including a stopping block positionable on the elongated portion of the cannula and distally of the sleeve stop, the stopping block configured to contact tissue and to prevent the sleeve stop from contacting tissue.

## Claims

1. A surgical access device comprising:
a cannula having a housing and an elongated portion extending distally from the housing, the elongated portion defining a channel extending therethrough, a distal portion of the elongated portion configured for engaging tissue; and
a sleeve stop configured for selective engagement with the elongated portion of the cannula, and configured to limit distal advancement of the cannula with respect to tissue, the sleeve stop being a unitary structure and including a body portion having a first section and a second section, the first section and the second section are interconnected by a living hinge, wherein the first section is configured to selectively engage the second section.

2. The surgical access device according to claim 1, wherein the first section of the body portion of the sleeve stop includes a finger, and wherein the second section of the body portion of the sleeve stop includes a receptacle configured for slidingly receiving the finger.

3. The surgical access device according to claim 2, wherein the finger includes a plurality of teeth.

4. The surgical access device according to claim 3, wherein the receptacle includes a plurality of teeth.

5. The surgical access device according to claim 4, wherein engagement between the plurality of teeth of the finger and the plurality of teeth of the receptacle helps maintain a size of an aperture defined between the first section of the body portion of the sleeve stop and the second portion of the body portion of the sleeve stop.

6. The surgical access device according to any preceding claim, wherein engagement between the first section of the body portion of the sleeve stop and the second portion of the body portion of the sleeve stop defines an aperture therebetween.

7. The surgical access device according to any preceding claim, wherein the sleeve stop is made from a single material.

8. The surgical access device according to claim 7, wherein the sleeve stop is made from plastic.

9. The surgical access device according to any preceding claim, further including a stopping block positionable on the elongated portion of the cannula and distally of the sleeve stop, the stopping block configured to contact tissue and to prevent the sleeve stop from contacting tissue.

10. A surgical access device comprising:
a cannula having a housing and an elongated portion extending distally from the housing, the elongated portion defining a channel extending therethrough, a distal portion of the elongated portion configured for engaging tissue; and
a sleeve stop configured for selective engagement with the elongated portion of the cannula, and configured to limit distal advancement of the cannula with respect to tissue, the sleeve stop including an adjustable member and a body portion having a first section and a second section, the first section being pivotable relative to the second section about a pivot, the first section and the second section defining an aperture therebetween for slidable reception of the cannula, the adjustable member configured to threadably engage a threaded aperture of the first section of the sleeve stop and a threaded aperture of the second section of the sleeve stop.

11. The surgical access device according to claim 10, wherein rotation of the adjustable member in a first direction relative to the first section of the sleeve stop causes a diameter of the aperture to increase, and wherein rotation of the adjustable member in a second direction relative to the first section of the sleeve stop causes the diameter of the aperture to decrease.

12. The surgical access device according to claim 10 or claim 11 wherein the adjustable member includes a wing nut.

13. The surgical access device according to any of claims 10 to 12, wherein the first section of the sleeve stop includes a semi-circular portion and an extension portion, and wherein the second section of the sleeve stop includes a semi-circular portion and an extension portion.

14. The surgical access device according to claim 13, wherein the threaded aperture of the first section of the sleeve stop is on the extension of the first section, and wherein the threaded aperture of the second section of the sleeve stop is on the extension of the second section.

15. The surgical access device according to any of claims 10 to 14 further including a stopping block positionable on the elongated portion of the cannula and distally of the sleeve stop, the stopping block configured to contact tissue and to prevent the sleeve stop from contacting tissue.
